(19) Europäisches Patentamt
European Patent Office
Office européen des brevets

(11) Veröffentlichungsnummer: **0 206 294 B1**

# (12) EUROPÄISCHE PATENTSCHRIFT

(45) Veröffentlichungstag der Patentschrift: **16.05.90**

(51) Int. Cl.⁵: **C07D 213/55**

(21) Anmeldenummer: **86108476.2**

(22) Anmeldetag: **20.06.86**

## (54) 3-Hydroxy-3-(2-Methyl-5-pyridyl)-propionsäurealkylester.

(30) Priorität: **20.06.85 CH 2614/85**

(43) Veröffentlichungstag der Anmeldung: **30.12.86 Patentblatt 86/52**

(45) Bekanntmachung des Hinweises auf die Patenterteilung: **16.05.90 Patentblatt 90/20**

(84) Benannte Vertragsstaaten: **AT BE CH DE FR GB IT LI LU NL SE**

(56) Entgegenhaltungen:
**EP-A- 0 003 677**
**EP-A- 0 070 185**

**Chem. Abstr. vol. 40, no. 16, 1946, Spalte 4723-4725**

(73) Patentinhaber: **LONZA AG, Gampel/Wallis(CH)**

(72) Erfinder: **Grayson, James Ian, Dr., 60 Albert Street, Western Hill Durham DH1 4RJ(GB)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40(DE)**

**EP 0 206 294 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 3-Hydroxy-3-(2-methyl-5-pyridyl)-propionsäurealkylester, ein Verfahren zu deren Herstellung sowie die Verwendung der erfindungsgemäßen Verbindungen zur Herstellung von 2-Methylpyridin-5-propionsäurealkylestern.

Die 2-Methylpyridin-5-propionsäurealkylester bilden wichtige Zwischenprodukte für eine Reihe von neuen Histamin $H_1$- und $H_2$-Antagonisten (Drugs of the Future, Vol. VII, No. 3, 156 ff. (1982).

Es ist bekannt, 3-Pyridinpropionsäureethylester durch Hydrierung von Nikotinoylacetat in Gegenwart eines Palladium/Bariumsulfatkatalysators in einer Ausbeute von 92% herzustellen [Graef et al, J. org. Chem. 11, 257 (1946)]. Wie eigene Versuche beweisen, läßt sich die Reaktion aber nicht auf 6-Methylnikotinoylacetat übertragen. Es ist außerdem bekannt, 2-Methylpyridin-5-propionsäurealkylester ausgehend von 6-Methylpyridin-3-carbaldehyd über eine dreistufige Synthese herzustellen (EP Appl. 0 003 677). Nachteilig ist aber, daß das Ausgangsprodukt das 6-Methylpyridin-3-carbaldeyd aus dem schwer erhältlichen und sehr teuren 2-Methyl-5-vinylpyridin hergestellt werden muß.

Die EP-A 0 070 185 beschreibt ebenfalls die Herstellung von 2-Methylpyridin-5-propionsäurealkylestern durch Umsetzung von 2-Methyl-5-vinylpyridin mit Kohlenmonoxid und dem entsprechenden Alkohol. Abgesehen davon, daß auch hier teures 2-Methyl-5-vinylpyridin verwendet werden muß, hat dieses Verfahren auch den Nachteil, daß neben dem gewünschten 3-Pyridinpropionsäurealkylester auch der isomere 2-Pyridinpropionsäurealkylester anfällt, der nur umständlich abzutrennen ist.

Aufgabe der vorliegenden Erfindung ist es daher, einen einfachen, technisch und wirtschaftlich gangbaren Weg zur Herstellung der 2-Methylpyridin-5-propionsäurealkylester zu finden.

Diese Aufgabe wird gelöst durch die Anwendung der neuen 3-Hydroxy-3-(2-methyl-5-pyridyl)-propionsäurealkylester der Formel

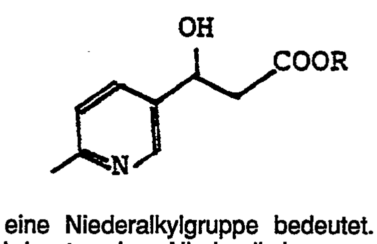

worin R eine Niederalkylgruppe bedeutet. Zweckmässig wird unter einer Niederalkylgruppe eine solche mit 1-4 C-Atomen verstanden die geradkettig oder verzweigt sein kann. Bevorzugt wird unter R aber eine Methyl- oder eine Ethylgruppe verstanden.

Die Herstellung der neuen Verbindungen erfolgt ausgehend von 5-Ethyl-2-methylpyridin, das durch Oxidation und Veresterung zu einem 6-Methylnikotinsäurealkylester umgesetzt wird, darauffolgend in Gegenwart eines Alkalimetallalkoholats mit einem Essigsäurealkylesters zum 6-Methylnikotinoylessigsäurealkylester umgesetzt wird und schliesslich in Gegenwart eines Hydrierkatalysators mit Wasserstoff zum 3-Hydroxy-3-(2-methyl-5-pyridyl)-propionsäurealkylester hydriert wird.

Erfindungsgemäss werden die neuen 3-Hydroxy-3-(2-methyl-5-pyridyl)-propionsäurealkylester zur Herstellung des 2-Methylpyridin-5-propionsäures alkylester mit Essigsäureanhydrid acetyliert. Das jeweilige Acetylierungsprodukt wird entweder isoliert oder direkt in situ in Gegenwart eines Hydrierkatalysators mit Wasserstoff zum Endprodukt hydrogenolysiert.

Die Oxidation von 5-Ethyl-2-methylpyridin zum 6-Methylnikotinsäurealkylester ist Gegenstand der CH-Anmeldung 2542/83 und wird folglich dieser entsprechend durchgeführt.

Die darauffolgende Umsetzung zum 6-Methylnikotinoylessigsäurealkylester erfolgt in Gegenwart eines Alkalimetallalkoholats mit dem entsprechenden Essigsäurealkylester unter an sich für Claisen-Kondensationen bekannten Bedingungen. Bevorzugt werden die Essigsäure $C_1$–$C_4$-alkylester verwendet. Es kommen zweckmäßig die dem Esterrest entsprechenden Natrium $C_1$–$C_4$-Alkoholate zur Anwendung. Der 6-Methylnikotinoylessigsäurealkylester kann isoliert und gereinigt werden, vorzugsweise wird er aber als Rohprodukt für die Hydrierung eingesetzt.

Die Hydrierung erfolgt zweckmäßig in Gegenwart eines üblichen Hydrierkatalysators.

Bevorzugt wird Palladium auf Kohle in einer Konzentration von 1% bis 10% Pd auf C vozugsweise 5% Pd auf C angewendet. Zweckmäßig wird unter einem Druck von 1 bis 15 bar vorzugsweise bei 7 bis 9 bar hydriert.

Als Lösungsmittel werden vorteilhaft die niederen aliphatischen Carbonsäuren, bevorzugt Essigsäure oder dem Esterrest entsprechende aliphatische Alkohole wie Methanol, Ethanol, Propanol oder Butanol verwendet. Es ist aber auch möglich in aprotischen Lösungsmitteln, wie z.B. in Toluol zu arbeiten.

Zweckmässig wird die Hydrierung in einem Temperaturbereich von 0° bis 100°C vorzugsweise bei 50° bis 80°C durchgeführt.

Nach beendeter Hydrierung, die je nach Druck zwischen 2 bis 12 h dauert, können nach üblicher Aufarbeitung die erfindungsgemässen 3-Hydroxy-3-(2-methyl-5-pyridyl)-propionsäurealkylester in guter Reinheit isoliert werden.

Zweckmässige Alkylester sind die Niederalkylester mit 1-4 C-Atomen. Als besonders bevorzugte Alkylester gelten der 3-Hydroxy-3-(2-methyl-5-pyridyl)-propionsäuremethylester und der 3-Hydroxy-3-(-2-methyl-5-pyridyl)-propionsäureethylester.

Diese neuen Verbindungen bilden die Zwischenprodukte für die Herstellung der 2-Methylpyridin-5-propionsäurealkylester. Sie können entweder direkt als Rohprodukt aus der vorgängigen Hydrierung oder speziell gereinigt für den folgenden Acetylierungsschritt eingesetzt werden.

Die Acetylierung erfolgt mit Essigsäureanhydrid, zweckmässig in Gegenwart von katalytischen Mengen eines tertiären Amins. Vorzugsweise wird 4-Dimethylaminopyridin in Mengen von 0,0001 Mol bis 0,01 Mol bezogen auf 1 Mol eingesetzten 3-Hy-

droxy-3-(2-methyl-5-pyridyl)-propionsäurealkylester eingesetzt.

Als Lösungsmittel kann das eingesetzte Essigsäureanhydrid selber verwendet werden, üblicherweise werden aber gegenüber Essigsäureanhydrid inerte Lösungsmittel wie z. B. Methylenchlorid, Chloroform, Tetrachlorkohlenstoff, Essigsäureester oder auch Toluol angewendet.

Die Reaktionstemperatur kann in einem Bereich von 0°-140°C variiert werden. Vorteilhaft wird aber zwischen 50°C und 80°c gearbeitet.

Das entstehende Acetylierungsprodukt kann isoliert und gereinigt werden, bevorzugt wird es aber in situ weiter zum Endprodukt hydrogenolysiert.

Die Hydrogenolyse erfolgt in Gegenwart eines üblichen Hydrierkatalysators vorteilhaft mit Palladium, das in einer Konzentration von zweckmässig 1 % bis 10 % auf dem Trägermaterial Kohle aufgebracht ist, mit Wasserstoff. Den Wasserstoffdruck wählt man zweckmässig in einen Bereich von 1 bis 15 bar vorteilhaft zwischen 7 bis 9 bar.

Das Lösungsmittel entspricht üblicherweise dem aus dem Acetylierungsschritt, vorteilhaft wird aber zusätzlich mit einer niederen organischen Carbonsäuren z. B. Essigsäure verdünnt. Die Reaktionstemperatur liegt zweckmässig zwischen 0° - 100°C vorteilhaft zwischen 50° - 80°C.

Nach beendeter Hydrogenolyse kann auf übliche Weise, z.B. durch anschliessende Neutralisation und Extraktion, das Endprodukt isoliert werden. Eine allfällige Reinigung kann durch Hochvakuumdestillation erfolgen. Mann kann nach diesem Verfahren die 2-Methylpyridin-5-propionsäurealkylester mit Gehalten von grösser als 97 % und in Ausbeuten von 50% bis 60% bezogen auf den 6-Methylnikotinsäurealkylester erhalten.

Den besonders vorteilhaften Zwischenprodukten 3-Hydroxy-3-(2-methyl-5-pyridyl)-propionsäuremethyl und -ethylestern entsprechend, werden bevorzugt der 2-Methylpyridin-5-propionsäuremethylester und der 2-Methylpyridin-5-propionsäureethylester hergestellt.

## Beispiele

### 1. Herstellung von 2-Methylpyridin-5-propionsäureethylester

#### 1.1 6-Methylnikotinoylessigsäureethylester

In einem 750 ml Sulfierkolben wurden 35,8 g (0,52 Mol) Natriumethylat und 200 ml Toluol vorgelegt und auf Rückfluss geheizt. Zur gerührten Suspension wurde während 7 Std. ein Gemisch von 55, 7 g (0,33 Mol) 6-Methylnikotinsäureethylester (hergestellt nach Schweizerischem Patentgesuch 2542/83) und 60,3 g (0,68 Mol) Essigsäureethylester zugetropft. Die Suspension wurde während 22 Std. beim Rückfluss gerührt und dann auf 20° C gekühlt. 200 ml Wasser und 25 ml konz. Salzsäure wurden zugegeben und die Phasen wurden getrennt. Die Wasserphase wurde mit Toluol extrahiert und die vereinigten organischen Phasen wurden eingedampft. Anschliessend wurde der Eindampfrückstand unter Vakuum destilliert. 25,9 g 6-Methylnikotinoylessigsäureethylester mit einem Kochpunkt von 112 - 130° C/0,2-0,4 mbar und einem Gehalt nach HPLC von 89,7 % wurde erhalten (Ausbeute 33,6 % bez. auf 6-Methylnikotinsäureethylester).

#### 1.2 3-Hydroxy-3-(2-methyl-5-pyridyl)-propionsäureethylester

10,0 g (0,048 Mol) 6-Methylnikotinoylessigsäureethylester (destilliert) wurden in 200 ml 95 %-igem Ethanol gelöst, mit 1 g 5 % Palladium auf Aktivkohle gemischt, und in einen 1 Liter Autoklav gefüllt. Der Autoklav wurde unter 10 bar Wasserstoff gestellt und bei 20° C gerührt. Nach 6 Stunden war die Hydrierung zu Ende. Der Autoklav wurde geöffnet und die Reaktionslösung wurde filtriert und eingedampft.

Es blieb 10,0 g 3-Hydroxy-3-(2-methyl-5-pyridyl)-propionsäureethylester zurück, das nach NMR und DC rein war. Die Ausbeute bezogen auf eingesetzten 6-Methylnikotinoylessigsäureethylester war 100 %. Ein Muster wurde destilliert (Kp. 130° C/0,2 mbar, Ausbeute der Destillation 94 %, Gehalt nach GC 97 %).

NMR: (in $CDCl_3$) δ 1,25 (t, J=7Hz, 3H), 2,5 (s, 3H), 2,7 (m, 2H). 4,15 (q, J=7Hz, 2H), 4,5 (br, 1H), 5,15 (dd, J=11 und 5 Hz, 1H), 7,15 (d, J=10Hz, 1H), 7,65 (dd, J=10 und 2Hz, 1H), 8,35 (d, J=2Hz, 1H).

IR: (dünne Schicht) $cm^{-1}$ 3200, 2980, 2920, 1730, 1600, 1565, 1490, 1440, 1370, 1280, 1250, 1200, 1160, 1035, 875, 835, 740.

MS: 209 ($M^+$, 8 %), 194 (5), 122 (100), 94 (23).

#### 1.3 2-Methylpyridin-5-propionsäureethylester

45,9 g (0,22 Mol) rohes 3-Hydroxy-3-(2-methyl-5-pyridyl)-propionsäureethylester wurde in 100 ml Essigsäureethylester gelöst und 50 ml (0,53 Mol) Essigsäureanhydrid und 0,1 g (0,82 mMol) 4-Dimethylaminopyridin wurden zugegeben. Die Lösung wurde während 6 Std. bei 20° C gerührt und dann eingedampft. Der Rückstand wurde in 50 ml Methylenchlorid und 50 ml 5 %-iger Natronlauge gelöst, die Phasen wurden getrennt und die organische Phase wurde eingedampft. 55,8 g rohes 3-Acetoxy-3-(2-methyl-5-pyridyl)-propionsäureethylester wurde erhalten. Dieses Rohprodukt wurde in 300 ml Essigsäure mit 3,0 g 5 % Palladium auf Kohle gelöst und in einen 1 Liter Autoklav gefüllt. Der Autoklav wurde unter 8 bar Wasserstoff gestellt und bei 70° C gerührt. Nach 6 Std. wurde die Hydrierung beendet. Der Autoklav wurde gekühlt und geöffnet, und die Lösung wurde filtriert und eingedampft. Der Rückstand wurde in 50 ml Wasser gelöst und mit Kaliumcarbonat auf pH 8 gebracht. Die Lösung wurde mit 3 mal 100 ml Methylenchlorid extrahiert und die organischen Extrakte wurden eingedampft. 42,5 g rohes 2-Methylpyridin-5-propionsäureethylester wurden erhalten. Das Rohprodukt wurde destilliert (Kp. 100-110° C/1,0 mbar). 22,0 g reines 2-Methylpyridin-5-propionsäureethylester wurde erhalten, mit einem GC-Gehalt von 92 %. Die Ausbeute bez. auf 6-Methylnikotinoylessigsäureethylester war 62,9 %.

2. Herstellung von 2-Methylpyridin-5-propionsäuremethylester

2.1. 3-Hydroxy-3-(2-methyl-5-pyridyl)-propionsäuremethylester

Aus 51,3 g (0,33 Mol) 6-Methylnikotinsäuremethylester (hergestellt nach der Methode vom Schweizerischen Patentgesuch 2542/83), 45,9 g (0,82 Mol) Natriummethylat und 49,6 g (0,67 Mol) Essigsäuremethylester wurde nach der allgemeinen Methode von Beispiel 1.1. 61,1 g rohes 6-Methylnikotinoylessigsäuremethylester hergestellt. 60,4 g dieses Rohproduktes wurden in 300 ml Essigsäure gelöst, 0,5 g 5 % Palladium auf Aktivkohle wurden zugegeben, und die Lösung wurde in einen 1 Liter Autoklav gefüllt. Die Hydrierung wurde unter einem Wasserstoffdruck von 8 bar und bei einer Temperatur von 65° C durchgeführt und brauchte 3,5 Std. Der Autoklav wurde gekühlt und geöffnet und die Lösung wurde filtriert und eingedampft. Der Rückstand wurde in 100 ml Wasser und 70 ml Methylenchlorid gelöst und mit 52,8 g 40 %-iger Natronlauge auf pH 7 gestellt. Die Phasen wurden getrennt, die Wasserphase wurde mit 2 x 70 ml Methylenchlorid extrahiert und die organischen Extrakte wurden eingedampft. 47,8 g roher Alkohol wurden produziert. 46,6 g Rohprodukt wurden aus 45 ml Toluol umkristallisiert. Das getrocknete Reinprodukt hatte einen Gehalt nach GC von 95,4 % und einen Schmelzpunkt von 74-76° C. Die Ausbeute von 35,1 g entspricht 53,7 % bez. auf eingesetzten 6-Methylnikotinsäuremethylester.
NMR: ($CDCl_3$): δ 2,5 (s, 3H), 2,7 (m, 2H), 3,7 (s, 3H), 4,0 (br, 1H), 5,15 (dd, J=11 und 6Hz, 1H), 7,1 (d, J=9Hz, 1H), 7,65 (dd, J=9 und 2Hz, 1H), 8,4 (d, J=2Hz, 1H).
IR: (KBR) $cm^{-1}$ 3460, 3140, 3040, 2970, 2840, 1735, 1605, 1495, 1440, 1395, 1355, 1325, 1280, 1230, 1205, 1165, 1075, 1030, 980, 930, 910, 890, 860, 740.
MS: 195 ($M^+$, 10 %), 180 (5), 122 (100), 94 (32).

2.2 2-Methylpyridin-5-propionsäuremethylester

80,0 g (0,35 Mol) umkristallisiertes 3-Hydroxy-3-(2-methyl-5-pyridyl)-propionsäuremethylester wurden in 100 ml Toluol gelöst. 60,0 g (0,588 Mol) Essigsäureanhydrid und 0,1 g (0,0009 Mol) 4-Dimethylaminopyridin wurden zugegeben und die Lösung wurde während 1 Std. bei 60° C gerührt. Dann wurden 20 ml Methanol zugegeben. Nach 15 Min. wurde mit 250 ml Essigsäure verdünnt, 1,5 g 5 % Palladium auf Aktivkohle wurden zugegeben, und die Lösung wurde in den Autoklav gefüllt. Die Hydrierung wurde unter 8 bar Wasserstoff bei 65° C während 5,5 Std. durchgeführt. Am Ende der Hydrierung wurde der Autoklav gekühlt und die Lösung wurde filtriert und eingedampft. Der Eindampfrückstand wurde in 100 ml Wasser und 100 ml Methylenchlorid gelöst und mit 87,9 g 40 %-iger Natronlauge auf pH 7 gestellt. Die Phasen wurden getrennt, die wässrige Phase wurde mit Methylenchlorid extrahiert und die vereinigten organischen Extrakte wurden eingedampft. 71, 5 g rohes 2-Methylpyridin-5-propionsäuremethylester wurde erhalten. (Gehalt nach GC 91,5 %, Ausbeute bezogen auf eingesetzten 3-Hydroxy-3-(2-methyl-5-pyridyl)-propionsäuremethylester 92,1 %). Das Produkt wurde destilliert, um 97 %-iges Produkt mit einem Kochpunkt von 100-113° C/1-2 mbar zu erhalten (Ausbeute an destilliertem Produkt bezogen auf eingesetzten 3-Hydroxy-3-(2-methyl-5-pyridyl)-propionsäuremethylester 88,5 %).

3. 2-Methylpyridin-5-propionsäuremethylester

Nach der Methode von Beispiel 2.1. wurde aus 51,3 g (0,33 Mol) 6-Methylnikotinsäuremethylester 48,0 g rohes 3-Hydroxy-3-(2-methyl-5-pyridyl)-propionsäuremethylester hergestellt. Dieses Rohprodukt wurde nicht umkristallisiert sondern mit 35,8 g Essigsäureanhydrid und 50 mg (0.41 mMol) 4-Dimethylaminopyridin in 50 ml Methylenchlorid gelöst und beim Rückfluss (40° C) während 1,5 Std. gerührt. 10 ml Methanol wurden zugegeben und nach 0,5 Std. wurde die Lösung eingedampft. Die rohe Acetoxyverbindung wurde in 300 ml Essigsäure in gegenwart von 4 g 5 % Palladium auf Aktivkohle hydriert.

Die Hydrierung dauerte 7 Std. bei einer Temperatur von 50°C und einem Wasserstoffdruck von 5 bar. Der 2-Methylpyridin-5-propionsäuremethylester wurde aus dem Reaktionsgemisch durch Eindampfen und Extraktion wie in Beispiel 2,2 isoliert. Es wurden 39,1 g rohes 2-Methylpyridin-5-propionsäuremethylester mit einem Gehalt nach GC von 83,1 % produziert, was einer Gesamtausbeute bez. auf 6-Methylnikotinsäuremethylester von 56,0 % entsprach.

4. 3-Hydroxy-3-(2-methyl-5-pyridyl)-propionsäuremethylester

Aus 51,3 g (0,33 Mol) 6-Methylnikotinsäuremethylester, 37,3 g (0,67 Mol) Natriummethylat und 49,6 g (0,67 Mol) Essigsäuremethylester wurde nach der allgemeinen Methode von Beispiel 1.1. 49,7 g rohes 6-Methylnikotinoylessigsäuremethylester hergestellt. 49,1 g dieses Produktes wurde mit 2,0 g 5 % Palladium auf Kohle in 300 ml Toluol hydriert.

Die Hydrierung brauchte 7 Std. bei einem Druck von 8 bar Wasserstoff und einer Temperatur von 50° C. Nach der Hydrierung wurde die Lösung filtriert und eingedampft. Das Rohprodukt wurde aus 40 ml Toluol umkristallisiert. Es wurden 21,0 g 3-Hydroxy-3-(2-methyl-5-pyridyl)-propionsäuremethylester hergestellt mit einem Gehalt nach GC von 95,5 %.

Die Ausbeute bez. auf 6-Methylnikotinsäuremethylester betrug 34,7 %.

5. 3-Acetoxy-3-(2-methyl-5-pyridyl)-propionsäuremethylester

10 g (0.05 Mol) 3-Hydroxy-3-(2-methyl-5-pyridyl)-propionsäuremethylester und 6,6 g (0.065 Mol) Essigsäureanhydrid wurden ohne Lösungsmittel auf Rückfluss (140° C) während 2 Std. geheizt. Die Lösung wurde gekühlt, in 50 ml Wasser gegossen und mit 8 ml 25 %-iger Ammoniaklösung auf pH 8 gestellt. Das Produkt wurde durch Extraktion mit 3 x

50 ml Methylenchlorid und Eindampfen der organischen Extrakte isoliert.

11,2 g (92 %) der Acetoxy-Verbindung wurden erhalten.

6. 3-Acetoxy-3-(2-methyl-5-pyridyl)-propionsäureethylester

4,3 g ( 0,021 Mol) 3-Hydroxy-3-(2-methyl-5-pyridyl)-propionsäureethylester, 3,0 g (0,03 Mol) Essigsäureanhydrid, 2,7 g (0,034 Mol) Pyridin und 0,17 g (0,0017 Mol) 4-Dimethylaminopyridin wurden bei 0° C während 24 Std. gerührt. Die Lösung wurde mit 25 ml 5 %-iger Natronlauge gemischt und mit 2 x 25 ml Methylenchlorid extrahiert.

Die organischen Extrakte wurden eingedampft; der Rückstand (4,7 g) enthielt 3-Acetoxy-3-(2-methyl-5-pyridyl)-propionsäureethylester (Ausbeute 90,4 %).

7. Vergleichsversuch
Hydrierung von 6-Methylnikotinoylessigsäureethylester nach der Methode von Graef et al. J. Org. Chem. 11 257 (1946).

5 g (0,026 Mol) 6-Methylnikotinoylessigsäuremethylester wurden in 40 ml Essigsäure gelöst, und 0,3 g 5 % Palladium auf Bariumsulfat und 3 Tropfen 60 %-ige Perchlorsäure wurden zugegeben. Die Reaktion wurde unter 1 bar $H_2$ bei 25° C hydriert. Es wurde keine Reaktion festgestellt. 0,3 g Katalyt und 3 Tropfen Perchlorsäure wurden zugegeben und die Hydrierung wurde bei 80° C unter 1 bar $H_2$ fortgesetzt. Nach 4 Std. wurde die Lösung filtriert und eingedampft.

Das Produkt wurde durch Neutralisation mit wässrigem Kaliumcarbonat und Extraktion mit Chloroform isoliert. 5,3 g Rohprodukt wurden erhalten und wurden von 2-Methylpyridin-5-propionsäureethylester wurden erhalten.

Das Hauptprodukt (63 % Ausbeute) war 3-Hydroxy-3-(2-methyl-5-pyridyl)-propionsäureethylester).

**Patentansprüche**

1. 3-Hydroxy-3-(2-methyl-5-pyridyl)-propionsäurealkylester der Formel

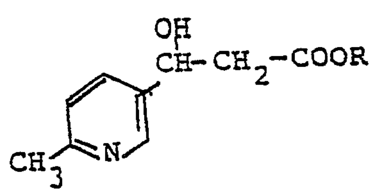

worin R eine $C_{1-4}$-alkylgruppe bedeutet.

2. 3-Hydroxy-3-(2-methyl-5-pyridyl)-propionsäuremethylester der Formel

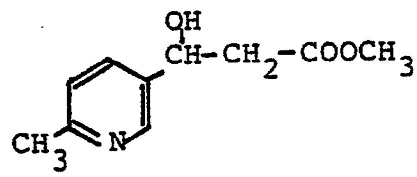

3. 3-Hydroxy-3-(2-methyl-5-pyridyl)-propionsäureethylester der Formel

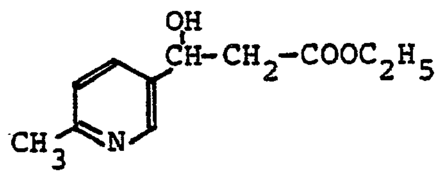

4. Verfahren zur Herstellung von Verbindungen nach Patentansprüchen 1 bis 3 aus 5-Ethyl-2-methylpyridin, das zuerst durch Oxydation und Veresterung in einen 6-Methylnikotinsäurealkylester überführt wird, dadurch gekennzeichnet, dass der 6-Methylnikotinsäurealkylester in Gegenwart eines Alkalimetallalkoholats mit einem Essigsäurealkylester zum 6-Methylnikotinoylessigsäurealkylester umgesetzt wird und weiter in Gegenwart eines Hydrierkatalysators mit Wasserstoff hydriert wird.

5. Verfahren nach Patentanspruch 4, dadurch gekennzeichnet, dass die Natrium-($C_1$-$C_4$)-alkoholate zur Anwendung kommen.

6. Verfahren nach Patentansprüchen 4 und 5, dadurch gekennzeichnet, dass die Essigsäure-($C_1$-$C_4$)-alkylester verwendet werden.

7. Verfahren nach Patentansprüchen 4 bis 6, dadurch gekennzeichnet, dass als Katalysator für die Hydrierung Palladium auf Kohle angewendet wird.

8. Verfahren nach Patentansprüchen 4 bis 7, dadurch gekennzeichnet, dass die Hydrierung unter einem Druck von 1 bis 15 bar durchgeführt wird.

9. Verfahren nach Patentansprüchen 4 bis 8, dadurch gekennzeichnet, dass die Hydriertemperatur zwischen 0°C und 100°C gewählt wird.

10. Verwendung der 3-Hydroxy-3-(2-methyl-5-pyridyl)-propionsäurealkylester nach Ansprüchen 1-3 zur Herstellung von 2-Methylpyridin-5-propionsäurealkylestern, dadurch gekennzeichnet, daß man mit Essigsäurenanhydrid acetyliert und das Acetylierungsprodukt entweder isoliert, oder es in situ, ohne Isolation in Gegenwart eines Hydrierkatalysators, mit Wasserstoff zum Endprodukt hydrogenolysiert.

11. Verwendung der Verbindungen nach Patentanspruch 10 zur Herstellung von 2-Methylpyridin-5-propionsäurealkylestern, dadurch gekennzeichnet, daß die Acetylierung in Gegenwart eines tertiären Amins als Katalysator erfolgt.

12. Verwendung der Verbindungen nach Patentansprüchen 10 bis 11 zur Herstellung von 2-Methylpyridin-5-propionsäurealkylestern, dadurch gekennzeichnet, daß die Acetylierung in Gegenwart von 4-Dimethylaminopyridin als Katalysator erfolgt.

13. Verwendung der Verbindungen nach Patentansprüchen 11 bis 12 zur Herstellung von 2-Methyl-

pyridin-5-propionsäurealkylestern, dadurch gekennzeichnet, daß die Acetylierung bei Temperaturen um 0°–140°C durchgeführt wird.

14. Verwendung der Verbindungen nach Patentansprüchen 11 bis 13 zur Herstellung von 2-Methyl-pyridin-5-propionsäurealkylestern, dadurch gekennzeichnet, daß als Hydrierkatalysator für die Hydrogenolyse Palladium auf Kohle verwendet wird.

15. Verwendung der Verbindungen nach Patentansprüchen 11 bis 14 zur Herstellung von 2-Methyl-pyridin-5-propionsäurealkylestern, dadurch gekennzeichnet, dass die Hydrogenolyse unter einem Druck von 1 bis 15 bar durchgeführt wird.

16. Verwendung der Verbindungen nach Patentansprüchen 11 bis 15 zur Herstellung von 2-Methyl-pyridin-5-propionsäurealkylestern, dadurch gekennzeichnet, dass die Hydrogenolyse bei Temperaturen von 0°C bis 100°C durchgeführt wird.

## Claims

1. 3-Hydroxy-3-(2-methyl-5-pyridyl) propionic acid alkyl ester of the formula

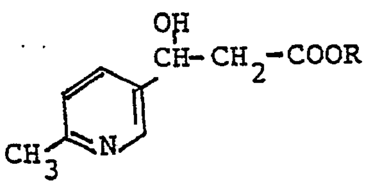

wherein R is an alkyl group having 1 to 4 carbon atoms.

2. 3-Hydroxy-3-(2-methyl-5-pyridyl) propionic acid methyl ester of the formula

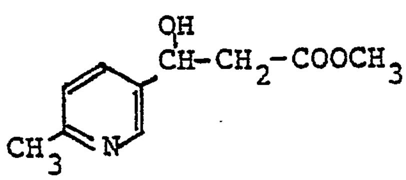

3. 3-Hydroxy-3-(2-methyl-5-pyridyl) propionic acid ethyl ester of the formula

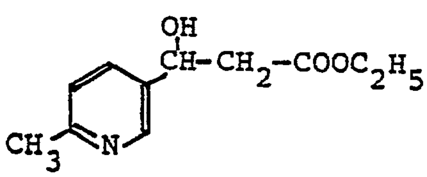

4. A method of preparing compounds according to any one of Claims 1 to 3 from 5-ethyl-2-methyl pyridine which is first converted into a 6-methyl nicotinic acid alkyl ester through oxidation and esterification, characterized in that said 6-methyl nicotinic acid alkyl ester is reacted with acetic acid alkyl ester in the presence of an alkali metal alcoholate to form 6-methylnicotinoyl acetic acid alkyl ester, and further hydrogenated with hydrogen in the presence of a hydrogenation catalyst.

5. The method according to Claim 4 wherein there is used a sodium-($C_1$–$C_4$)-alcoholate.

6. The method according to Claims 4 and 5 wherein there is used an acetic acid-($C_1$–$C_4$)-alkyl ester.

7. The method according to any one of Claims 4 to 6 wherein palladium on carbon is used as a hydrogenation catalyst.

8. The method according to any one of Claims 4 to 7 wherein hydrogenation is conducted under a pressure of from 1 to 15 bar.

9. The method according to any one of Claims 4 to 8 wherein the hydrogenation temperature is between 0° and 100°C.

10. Use of the 3-hydroxy-3-(2-methyl-5-pyridyl) propionic acid alkyl ester according to Claims 1 to 3 for the production of 2-methylpyridino-5-propionic acid alkyl ester, characterized in that acetylation is effected with acetic anhydride and the acetylation product is either isolated or hydrogenolyzed in situ, without isolation, using hydrogen in the presence of a hydrogenation catalyst to produce the final product.

11. Use of the compounds according to Claim 10 for the production of 2-methylpyridino-5-propionic acid alkyl ester characterized in that acetylation is conducted in the presence of a tertiary amine as a catalyst.

12. Use of the compounds according to Claims 10 and 11 for the production of 2-methylpyridino-5-propionic acid alkyl ester characterized in that acetylation is conducted in the presence of 4-diamethylamino pyridine as a catalyst.

13. Use of the compounds according to any one of Claims 11 to 12 for the production of 2-methyl-pyridino-5-propionic acid alkyl ester characterized in that acetylation is conducted at a temperature between 0° and 140°C.

14. Use of the compounds according to any one of Claims 11 to 13 for the production of 2-methyl-pyridino-5-propionic acid alkyl ester characterized in that palladium on carbon is used as a hydrogenation catalyst for hydrogenolysis.

15. Use of the compounds according to any one of Claims 11 to 14 for the production of 2-methyl-pyridino-5-propionic acid alkyl ester characterized in that hydrogenolysis is conducted under a pressure of from 1 to 15 bar.

16. Use of the compounds according to any one of Claims 11 to 15 for the production of 2-methyl-pyridino-5-propionic acid alkyl ester characterized in that hydrogenolysis is conducted at temperatures between 0° and 100°C.

## Revendications

1. Esters alcoyliques de l'acide 3-hydroxy-3-(2-méthyl-5-pyridyl)-propionique de formule

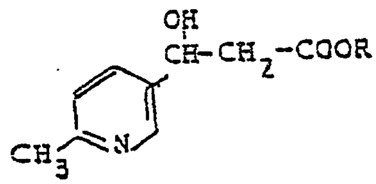

dans laquelle R signifie un groupe alcoyle en $C_{1-4}$.

2. 3-hydroxy-3-(2-méthyl-5-pyridyl)-propionate de méthyle de formule

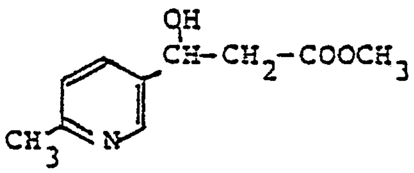

3. 3-hydroxy-3-(2-méthyl-5-pyridyl)-propionate d'éthyle de formule

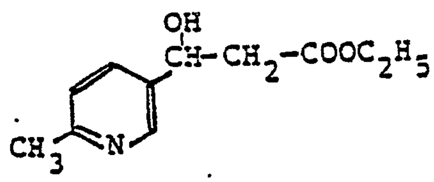

4. Procédé pour la préparation des composés selon les revendications 1 à 3 à partir de la 5-éthyl-2-méthylpyridine qui est d'abord transformée par oxydation et estérification en un ester alcoylique de l'acide 6-méthylnicotinique, caractérisé en ce qu'on fait réagir l'ester alcoylique de l'acide 6-méthylnicotinique en présence d'un alcoolate de métal alcalin avec un ester alcoylique de l'acide acétique pour donner l'ester alcoylique de l'acide 6-méthylnicotinoylacétique et on poursuit en hydrogénant au moyen d'hydrogène, en présence d'un catalyseur d'hydrogénation.

5. Procédé selon la revendication 4, caractérisé en ce qu'on a recours aux alcoolates de sodium en $C_1$–$C_4$.

6. Procédé selon les revendications 4 et 5, caractérisé en ce que les esters alcoyliques en $C_1$–$C_4$ de l'acide acétique sont utilisés.

7. Procédé selon les revendications 4 à 6, caractérisé en ce qu'on utilise comme catalyseur pour l'hydrogénation, du palladium sur charbon.

8. Procédé selon les revendications 4 à 7, caractérisé en ce que l'hydrogénation est effectuée sous une pression de 1 à 15 bars.

9. Procédé selon les revendications 4 à 8, caractérisé en ce que la température d'hydrogénation est choisie entre 0° et 100°C.

10. Utilisation des esters alcoyliques de l'acide 3-hydroxy-3-(2-méthyl-5-pyridyl)-propionique selon les revendications 1 à 3 pour la préparation d'esters alcoyliques de l'acide 2-méthylpyridine-5-propionique, caractérisée en ce qu'on acétyle au moyen d'anhydride acétique et en ce que soit on isole le produit d'acétylation, soit on l'hydrogénolyse in situ sans isolement, en présence d'un catalyseur d'hydrogénation, au moyen d'hydrogène pour donner le produit final.

11. Utilisation des composés selon la revendication 10 pour la préparation d'esters alcoyliques de l'acide 2-méthylpyridine-5-propionique, caractérisée en ce que l'acétylation a lieu en présence d'une amine tertiaire comme catalyseur.

12. Utilisation des composés selon les revendications 10 à 11 pour la préparation d'esters alcoyliques de l'acide 2-méthylpyridine-5-propionique, caractérisée en ce que l'acétylation a lieu en présence de 4-diméthylaminopyridine comme catalyseur.

13. Utilisation des composés selon les revendications 11 à 12 pour la préparation d'esters alcoyliques de l'acide 2-méthylpyridine-5-propionique, caractérisée en ce que l'acétylation est effectuée à des températures aux environs de 0°–140°C.

14. Utilisation des composés selon les revendications 11 à 13 pour la préparation d'esters alcoyliques de l'acide 2-méthylpyridine-5-propionique, caractérisée en ce qu'on utilise comme catalyseur d'hydrogénation pour l'hydrogénolyse du palladium sur charbon.

15. Utilisation des composés selon les revendications 11 à 14 pour la préparation d'esters alcoyliques de l'acide 2-méthylpyridine-5-propionique, caractérisée en ce que l'hydrogénolyse est effectuée sous une pression de 1 à 15 bars.

16. Utilisation des composés selon les revendications 11 à 15 pour la préparation d'esters alcoyliques de l'acide 2-méthylpyridine-5-propionique, caractérisée en ce que l'hydrogénolyse est effectuée à des températures de 0°C à 100°C.